# EUROPÄISCHE PATENTSCHRIFT

(11) **EP 1 575 961 B1**
(45) Veröffentlichungstag und Bekanntmachung des Hinweises auf die Patenterteilung: **20.06.2007**
(21) Anmeldenummer: 03780117.2
(22) Anmeldetag: 04.12.2003
(51) Int. Cl.: C07D 495/04, C07D 333/38

(54) **VERFAHREN ZUR DECARBOXYLIERUNG VON DICARBONSÄUREN**
METHOD FOR THE DECARBOXYLATION OF DICARBOXYLIC ACIDS
PROCEDE DE DECARBOXYLATION D'ACIDES DICARBOXYLIQUES

(30) Priorität: 16.12.2002 DE 10258588
(43) Veröffentlichungstag der Anmeldung: 21.09.2005
(73) Patentinhaber: Saltigo GmbH, 51369 Leverkusen (DE)
(72) Erfinder: BUCHHOLZ, Sigurd, 50825 Köln (DE); KLAUSENER, Alexander, 50259 Pulheim (DE); LANGER, Reinhard, 47918 Tönisvorst (DE); MLECZKO, Leslaw, 44801 Bochum (DE); RAUCHSCHWALBE, Günter, 51375 Leverkusen (DE)
(74) Vertreter: Wichmann, Birgid
(86) Internationale Anmeldenummer: PCT/EP2003/013679
(87) Internationale Veröffentlichungsnummer: WO 2004/055023

(56) Entgegenhaltungen:
- EP-A- 1 142 888
- DE-A- 10 029 078
- OVERBERGER C G ET AL: "THE PREPARATION OF 3,4-DIMETHOXY-2,5-DICARBETHOXYTHIOPHENE. 3,4-DIMETHOXYTHIOPHENE" JOURNAL OF THE AMERICAN CHEMICAL SOCIETY, AMERICAN CHEMICAL SOCIETY, WASHINGTON, DC, US, Bd. 73, 1951, Seiten 2956-2957, XP001005623 ISSN: 0002-7863
- MERZ A ET AL: "IMPROVED PREPARATION OF 3,4-DIMETHOXYTHIOPHENE" JOURNAL FUR PRAKTISCHE CHEMIE, CHEMIKER ZEITUNG, WILEY VCH, WEINHEIM, DE, Bd. 338, 1996, Seiten 672-674, XP001000173 ISSN: 1436-9966 in der Anmeldung erwähnt

## Beschreibung

Die Erfindung betrifft ein Verfahren zur thermischen Decarboxylierung von Dicarbonsäuren, insbesondere die Decarboxylierung von Dialkoxythiophen- und Alkylendioxythiophen-dicarbonsäuren ohne die Zugabe eines zusätzlichen Lösungsmittels, ggf. unter Zugabe eines inerten Gases.

Inerte Gase im Sinne dieser Erfindung sind solche Gase, die unter den verwendeten Bedingungen keine Reaktion mit der Dicarbonsäure eingehen.

Zur Decarboxylierung von Mono- und Polycarbonsäuren sind verschiedene Verfahren bekannt. Nach US-A 2,453,103 wird die thermische Decarboxylierung von 3,4-Dimethoxythiophen-2,5-dicarbonsäure unter Zugabe eines pulverförmigen Kupferkatalysators durchgeführt. Ähnliche Verfahrenswege für die Decarboxylierung von Dialkoxythiophen- und Alkylendioxythiophen-dicarbonsäuren werden z.B. von Coffey et al. (, thermische Decaboxylierung des geschmolzenen Materials, in: Synthetic Communications, 26(11), 2205-2212, 1996), von Stéphan et al (Decaboxylierung bei 180°C unter Zugabe von Chrom(III)-Kupfer(II)-oxid, in: Journal of Electroanalytical Chemistry, 443, 217-226, 1998) oder Merz und Rehm (ohne Katalysator oder Verdünnungsmittel, in: Journal für Praktische Chemie, 338, 672-674, 1996). Die bei diesen Verfahren verwendeten Bedingungen orientieren sich meist an der thermischen Instabilität der Verbindungen. So wird die Decarboxylierung vorteilhaft in einem Lösungsmittel mit Zugabe eines die Decarboxylierung beschleunigenden Katalysators durchgeführt. Die Decarboxylierung von 3,4-Ethylendioxythiophen-2,5-dicarbonsäure zu 3,4-Ethylendioxythiophen wird so in einem organischen Lösungsmittel (beispielsweise Tetrahydrothiophen-1,1-dioxid) unter Zugabe eines Kupfercarbonats als Katalysator bei Temperaturen von 100-200°C und Drücken von 800-1200 hPa durchgeführt. Bei dieser Form der Verfahrensführung wird zunächst das gesamte Edukt decarboxyliert und dann das Produkt aus dem Lösungsmittel bei reduziertem Druck abdestilliert.

Neben der 3,4-Ethylendioxythiophen-2,5-dicarbonsäure enthält das Edukt jedoch immer geringe Mengen an Nebenprodukten, die in vorigen Syntheseschritten gebildet werden. Diese Nebenprodukte reichem sich im Lösungsmittel an und begrenzen die Wiederverwendbarkeit von Lösungsmittel und Katalysator. Es bilden sich Cu-haltige Abfälle, die aufwendig entsorgt werden müssen. Darüber hinaus kann das beschriebene Verfahren zur Decarboxylierung von 3,4-Ethyledioxythiophen-2,5-dicarbonsäure, dass den Stand der Technik darstellt, nur absatzweise betrieben werden. Bei der rein thermischen Decarboxylierung verbleibt ein großer Teil des Produktes im Reaktionsraum und es kommt aufgrund der erhöhten Temperaturen zur verstärkten Bildung von Nebenprodukten, die die Qualität stark beeinträchtigen oder aber eine aufwendige Nachreinigung notwendig machen.

Die Aufgabe der vorliegenden Erfindung besteht darin, eine Verfahrensführung für die Decarboxylierung der 3,4-Ethylendioxythiophen-2,5-dicarbonsäure und ähnlicher Dicarbonsäuren zu entwickeln, die eine kontinuierliche oder quasi-kontinuierliche Betriebsweise bei gleichzeitiger Minimierung des Einsatzes von Betriebsstoffen (Lösungsmittel und Katalysator) ermöglicht.

Gegenstand der Erfindung, durch den die Aufgabe gelöst wird, ist ein Verfahren zur thermischen Decarboxylierung von Dicarbonsäuren, insbesondere von 3,4-Ethylendioxythiophen-2,5-dicarbonsäure als Edukt, dadurch gekennzeichnet, dass man das Edukt als Feststoff einsetzt und/oder die Reaktion in Anwesenheit einer Vielzahl von Wirbelschichtkörpern durchführt, wobei man die Reaktion in Abwesenheit von Lösungsmitteln durchführt und das bei der Reaktion entstehende Decarboxylierungsprodukt, insbesondere das 3,4-Ethylendioxythiophen, gasförmig aus der Reaktionszone abführt.

Bevorzugt wird die Decarboxylierung bei einer, Temperatur von 100 bis 600°C, bevorzugt 100 bis 500°C, besonders bevorzugt 150 bis 400°C durchgeführt.

Überraschenderweise zeigte sich in Versuchen zur rein thermischen Decarboxylierung von 3,4-Ethylendioxythiophen-2,5-dicarbonsäure in einer Wirbelschicht, dass 3,4-Ethylendioxythiophen in sehr hohen Selektivitäten auch in Abwesenheit eines Lösungsmittels durch die alleinige heterogene Umsetzung des Eduktfeststoffs erhalten werden kann. Es zeigte sich, dass das gebildete 3,4-Ethylendioxythiophen bei der zur Decarboxylierung notwendigen Temperatur einen genügend hohen Dampfdruck aufweist, um gasförmig mit einem Inertgasstrom ausgetragen und durch Kühlung abgeschieden werden zu können. Auf diese Art und Weise wird der Einsatz von Betriebsstoffen minimiert und ein kontinuierlicher bzw. quasi-kontinuierlicher Betrieb ermöglicht.

Das erfindungsgemäße Verfahren kann in verschiedenen Reaktortypen durchgeführt werden, solange das durch die Decarboxylierung gebildete Produkt, z.B. 3,4-Ethylendioxythiophen, in gasförmiger Form aus dem Reaktor ausgetragen wird. Beispielhaft seien hier Festbettreaktoren, Wanderbettreaktoren, Reaktoren mit einem blasenbildenden, turbulenten oder durchstrahlten Wirbelbett, intern oder extern zirkulierende Wirbelbetten genannt. Es ist auch möglich, das Edukt , z.B. die 3,4-Ethylendioxythiophen-2,5-dicarbonsäure in einen mit Wirbelschichtkörpern gefüllten Reaktor zu geben, der beispielsweise unter die oben genannten Klassen fällt.

Das Verfahren wird insbesondere kontinuierlich in einer blasenbildenden, oder turbulenten oder durchstrahlten Wirbelschicht, oder in einem intern oder extern zirkulierten Wirbelbett durchgeführt.

Die Reaktion wird besonders bevorzugt in Gegenwart eines inerten Hilfsgases, insbesondere ausgewählt aus der Reihe Edelgase, Stickstoff, Wasserdampf, Kohlenmonoxid oder Kohlendioxid oder einer Mischung verschiedener derartiger inerter Hilfsgase durchgeführt.

Als inerte Gase kommen alle Gase in Frage, die unter den gewählten Reaktionsbedingungen nicht mit dem Edukt oder Produkt reagieren, geeignete Inertgase sind beispielsweise Edelgase, Stickstoff, Wasserdampf, Kohlenmonoxid oder Kohlendioxid. Es ist möglich, das erfindungsgemäße Verfahren zur Decarboxylierung von 3,4-Ethylendioxythiophen-2,5-dicarbonsäure unter Zugabe eines inerten Gases oder einer Mischung mehrerer inerter Gase in beliebiger Kombination durchzuführen.

Die Temperatur kann wie beschrieben im Temperaturbereich von 100°C bis 600°C variiert werden. Sie muss jedoch so hoch sein, dass die Decarboxylierung des Edukts z.B. von 3,4-Ethylendioxythiophen-2,5-dicarbonsäure gewährleistet ist und darf die Zersetzungstemperatur des 3,4-Ethylendioxythiophen-2,5-dicarbonsäure bzw. 3,4-Ethylendioxythiophen nicht überschreiten.

Bevorzugt wird die Reaktion in einem Wirbelschicht- oder Wirbelbettreaktor durchgeführt, in dem Wirbelschichtkörper eingesetzt werden mit einem mittleren Durchmesser (Zahlenmittel), der größer als der Partikeldurchmesser der Dicarbonsäure ist.

Die Wirbelschichtkörper weisen insbesondere bevorzugt eine Feststoffdichte ρₛ von 0,5 g·cm⁻³ < ρₛ < 6 g·cm⁻³ auf.

Die Wirbelschichtkörper können auch bevorzugt als Wärmeträger verwendet werden, die außerhalb der Reaktionszone vorgeheizt und durch die Reaktionszone zirkuliert werden.

Die Wirbelschichtkörper bestehen bevorzugt teilweise oder ganz aus einem katalytisch aktiven Material insbesondere Kupfer oder einem Kupfersalz, bevorzugt aus CuCO₃.

Das erfindungsgemäße Verfahren wird bevorzugt in einer Wirbelschicht durchgeführt. Dazu werden im Reaktionsraum Feststoffpartikel der 3,4-Ethylendioxythiophen-2,5-dicarbonsäure, im weiteren Partikel genannt, vorgelegt. Die Partikel können absatzweise oder auch kontinuierlich von außen zugeführt werden. Die Partikel bilden ein Festbett, durch das das zugeführte Gas eingeströmt wird. Die Einströmgeschwindigkeit des zugeführten Gases kann so eingestellt, werden dass das Festbett fluidisiert wird und sich eine Wirbelschicht ausbildet. Die entsprechende Vorgehensweise ist dem Fachmann an und für sich bekannt. Die Einströmgeschwindigkeit des zugeführten Gases muss dazu mindestens der Lockerungsgeschwindigkeit (auch Minimumfluidisierungsgeschwindigkeit umf) entsprechen. Unter Lockerungsgeschwindigkeit wird dabei die Geschwindigkeit verstanden, mit der ein Gas durch ein Bett aus Partikeln strömt und unterhalb derer das Festbett erhalten bleibt, d.h. unterhalb derer die Bettpartikel weitgehend unbewegt bleiben. Oberhalb dieser Geschwindigkeit beginnt die Fluidisierung des Betts, d.h. die Bettpartikel bewegen sich und es bilden sich erste Blasen. Beim Betrieb einer blasenbildenden Wirbelschicht wird die Gasgeschwindigkeit so gewählt, dass diese dem ein bis zehnfachen der Lockerungsgeschwindigkeit entspricht, bevorzugt dem ein bis siebenfachen, besonders bevorzugt dem ein- bis fünffachen.

Fällt das Edukt in einer sehr feinen Kornfraktion an, so überwiegen interpartikuläre Kräfte und die Feststoffe sind schwer zu handhaben. In diesem Fall kann es vorteilhaft sein, das Edukt 3,4-Ethylendioxythiophen-2,5-dicarbonsäure in ein Bett aus größeren Partikeln einzubringen. Liegt die mittlere Partikelgröße der 3,4-Ethylendioxythiophen-2,5-dicarbonsäure beispielsweise bei 0,1-50 µm, so kann die Handhabung in einem Bett mit Partikeln, die einen mittleren Partikeldurchmesser dP von 50 < dP < 350 µm aufweisen, wesentlich erleichtert werden. Diese größeren Bettpartikeln weisen bevorzugt eine Feststoffdichte ρs von 0,5 g·cm⁻³ <ρs 4 g·cm⁻³ auf. Diese beschriebenen Partikeln wirken dabei als Träger für die kleinen Partikeln, die an der Oberfläche haften. Darüber hinaus können die vorgelegte Bettpartikeln als Impuls- und Wärmeträger dienen und / oder ganz oder teilweise aus einem katalytisch aktiven Material bestehen. Als katalytisch aktive Materialien können beispielsweise Metalle, Metalloxide oder Metallsalze verwendet werden. Besonders bevorzugt werden Kupfer, Kupferoxide und Kupfersalze eingesetzt. Es können Vollmaterialien eingesetzt werden, die aktiven Komponenten können jedoch auch mit einem Träger gemischt oder aufgebracht werden. Der Einsatz reiner Komponenten oder von Gemischen ist denkbar. Der Eintrag von Edukt 3,4-Ethylendioxythiophen-2,5-dicarbonsäure kann beispielsweise über Schnecken, Injektoren oder ein Schleusensystem erfolgen. Eine weitere Form des Eintrags kann in dem Eindüsen von geschmolzener oder in mit Hilfe eines Lösungsmittels verdünnter 3,4-Ethylendioxythiophen-2,5-dicarbonsäure erfolgen.

Das Einbringen eines Katalysators in reiner Form, als Gemisch oder in geträgerter Form kann auch in Form von Einbauten in dem Reaktor erfolgen. Beispielhaft genannt seien starre oder flexible Einbauten, stab- und röhrenförmige Einbauten, Konstruktionen in Form von Lochblechen, Netzen, Gittern oder dreidimensionalen Strukturen sowie Packungen und die Verwendung von Formkörpern oder frei fliessenden Elementen geschehen.

Die Abtrennung von feinteiligem Eduktfeststoff aus dem den Reaktor verlassenden Gasstrom kann beispielsweise über einen Zyklon, einen Filter oder über einen Gaswäscher erfolgen. Bevorzugt ist eine Abtrennung über einen Zyklon und/oder einen Filter. Der gesammelte Eduktfeststoff wird vorteilhaft wieder in den Decarboxylierungsschritt zurückgeführt. Diese Rückführung kann beispielsweise durch eine interne oder externe Zirkulation erfolgen. Die Rückführung kann jedoch auch durch das Rückblasen und Abreinigen von Filtern erfolgen.

Ein bevorzugtes Verfahren ist dadurch gekennzeichnet, dass gegebenenfalls durch den Gasstrom aus der Reaktionszone ausgetragener Feststoff mit Hilfe eines Zyklons und/oder Filters vom Produkt abgetrennt wird.

Bevorzugt ist ebenfalls eine Variante des Verfahrens bei der vom Produktgasstrom abgetrennter, nicht umgesetzter Eduktfeststoff absatzweise oder kontinuierlich wieder in die Reaktionszone zurückgeführt wird.

Im Folgenden wird das erfindungsgemäße Verfahren anhand einiger Beispiele illustriert.

### Beispiele

### Beispiel 1 (Festbett):

In einem Glasreaktor (Durchmesser 16 mm, Gesamthöhe 400 mm) wurden 4 g getrocknete Rohware der 3,4-Ethylendioxythiophen-2,5-dicarbonsäure vorgelegt und im Stickstoffstrom auf eine maximale Reaktionstemperatur von 300°C aufgeheizt (Heizrate 2 °C/min). Über eine Zeit von 80 min wurde das Edukt abreagiert. Von den vorgelegten 4 g (1,7·10⁻² Mol) wurden 3,5 g (1,52·10⁻² Mol) abreagiert und zu einem geringen Anteil durch den Stickstoffstrom aus dem Reaktor ausgetragen. Es wurden in einer nachgeschalteten Kühlfalle 2,89 g eines Produktgemisches aus 3,4-Ethylendioxythiophen-2,5-dicarbonsäure und 3,4-Ethylendioxythiophen aufgefangen. Der durch HPLC bestimmte Gehalt an 3,4-Ethylendioxythiophen betrug > 90 Gew.-%.

### Beispiel 2 (Wirbelbett , Strahlschichtreaktor):

In einem Glasreaktor (Durchmesser 50 mm, Gesamthöhe 730mm, Konischer Gaseinlass mit einer Gasverteilerfritte 10 mm) wurden 120,0 g (0,52 mol) getrocknete Rohware der 3,4-Ethylendioxythiophen-2,5-dicarbonsäure vorgelegt und im Stickstoffstrom (Normalbedingungen) auf eine maximale Reaktionstemperatur von 320°C aufgeheizt (Heizrate 2°C/min). Über eine Zeit von 100 mm wurde das Edukt abreagiert. Von der Menge Rohware wurden 32,0 g (0,139 mol) abreagiert. In einer nachgeschalteten Kühlfalle wurden 16,6 g Produkt auskondensiert. Der Gehalt an 3,4-Ethylendioxythiophen betrug > 94 Gew.-%.

### Beispiel 3 "Particle Powder"-Wirbelschicht:

In einem Glasreaktor (Durchmesser 95 mm, Gesamthöhe 700 mm, Gasverteilerfritte 95 mm) mit zusätzlichem Gasverteiler wurden 3000 g Quarzsand mit einem Durchmesser von 160-250 mm vorgelegt und auf eine Reaktionstemperatur von 280°C im Quarzbett unter Fluidisierung mit Stickstoff aufgeheizt. Ein Gemisch aus 0,1351 mol 3,4-Ethylendioxythiophen-2,5-dicarbonsäure und basischem Kupfercarbonat im Massenverhältnis 1:1 wurde über eine Zeit von 58 Minuten in den mit Stickstoff fluidisierten Reaktor gegeben. Dort lief die Decarboxylierung ab und das Produkt wurde in mehreren hintereinander geschalteten Kühlfallen kondensiert. Die Ausbeute betrug Y = 83 mol.-% bei einer durch HPLC bestimmten Reinheit des Produktes von mehr als 94 Gew.-%. Der aus dem Reaktor ausgetragene und in einem Zyklon abgetrennte Feststoff kann wieder als Edukt eingesetzt werden.

## Patentansprüche

1. Verfahren zur thermischen Decarboxylierung von Dialkoxythiophen- und Alkylendioxythiophendicarbonsäuren, insbesondere von 3,4-Ethylendioxythiophen-2,5-dicarbonsäure als Edukt, **dadurch gekennzeichnet, dass** man das Edukt als Feststoff einsetzt und/oder die Reaktion in Anwesenheit einer Vielzahl von Wirbelschichtkörpern durchführt, wobei man die Reaktion in Abwesenheit von Lösungsmitteln durchführt und das bei der Reaktion entstehende Decarboxylierungsprodukt, insbesondere das 3,4-Ethylendioxythiophen, gasförmig aus der Reaktionszone abführt.

2. Verfahren nach Anspruch 1, **dadurch gekennzeichnet, dass** die Decarboxylierung bei einer Temperatur von 100 bis 600°C, bevorzugt 100 bis 500°C, besonders bevorzugt 150 bis 400°C durchgeführt wird.

3. Verfahren nach wenigstens einem der Ansprüche 1 bis 2, **dadurch gekennzeichnet, dass** das Verfahren kontinuierlich in einer blasenbildenden, oder turbulenten oder durchstrahlten Wirbelschicht, oder in einem intern oder extern zirkulierten Wirbelbett durchgeführt wird.

4. Verfahren nach wenigstens einem der Ansprüche 1 bis 3, **dadurch gekennzeichnet, dass** die Reaktion in Gegenwart eines inerten Hilfsgases, insbesondere ausgewählt aus der Reihe Edelgase, Stickstoff, Wasserdampf, Kohlenmonoxid oder Kohlendioxid oder einer Mischung verschiedener derartiger inerter Hilfsgase durchgeführt wird.

5. Verfahren nach einem der Ansprüche 1 bis 4, **dadurch gekennzeichnet, dass** die Reaktion in einem Wirbelschicht- oder Wirbelbettreaktor durchgeführt wird, in dem Wirbelschichtkörper eingesetzt werden mit einem mittleren Durchmesser (Zahlenmittel), der größer als der Partikeldurchmesser der Dicarbonsäure ist.

6. Verfahren nach Anspruch 5, **dadurch gekennzeichnet, dass** die Wirbelschichtkörper eine Feststoffdichte ρₛ von 0,5 g·cm⁻³ < ρₛ < 6 g·cm⁻³ aufweisen.

7. Verfahren nach einem der Ansprüche 1 bis 6, **dadurch gekennzeichnet, dass** die Wirbelschichtkörper als Wärmeträger verwendet werden die außerhalb der Reaktionszone vorgeheizt und durch die Reaktionszone zirkuliert werden teilweise oder ganz aus einem katalytisch aktiven Material bestehen.

8. Verfahren nach einem der Ansprüche 1 bis 7, **dadurch gekennzeichnet, dass** die Wirbelschichtkörper teilweise oder ganz aus einem katalytisch aktiven Material insbesondere Kupfer oder einem Kupfersalz, bevorzugt CuCO₃ bestehen.

9. Verfahren nach einem der Ansprüche 1 bis 8, **dadurch gekennzeichnet, dass** gegebenenfalls durch den Gasstrom aus der Reaktionszone ausgetragener Feststoff mit Hilfe eines Zyklons und/oder Filters vom Produkt abgetrennt wird.

10. Verfahren nach einem der Ansprüche 1 bis 9, **dadurch gekennzeichnet, dass** vom Produktgasstrom abgetrennter, nicht umgesetzter Eduktfeststoff absatzweise oder kontinuierlich wieder in die Reaktionszone zurückgeführt wird.

## Claims

1. Process for the thermal decarboxylation of dialkoxythiophenedicarboxylic and alkylenedioxythiophenedicarboxylic acids, in particular 3,4-ethylene-dioxythiophene-2,5-dicarboxylic acid, as starting material, **characterized in that** the starting material is used as a solid and/or the reaction is carried out in the presence of a plurality of fluidized-bed bodies, with the reaction being carried out in the absence of solvents and the decarboxylation product formed in the reaction, in particular 3,4-ethylenedioxythiphene, being discharged from the reaction zone in gaseous form.

2. Process according to Claim 1, **characterized in that** the decarboxylation is carried out at a temperature of from 100 to 600°C, preferably from 100 to 500°C, particularly preferably from 150 to 400°C.

3. Process according to at least one of Claims 1 and 2, **characterized in that** the process is carried out continuously in a bubble-forming or turbulent or jet-permeated fluidized bed or in an internally or externally circulating fluidized bed.

4. Process according to at least one of Claims 1 to 3, **characterized in that** the reaction is carried out in the presence of an inert auxiliary gas, in particular a gas selected from the group consisting of noble gases, nitrogen, water vapor, carbon monoxide and carbon dioxide and mixtures of various such inert auxiliary gases.

5. Process according to any of Claims 1 to 4, **characterized in that** the reaction is carried out in a fluidized-bed reactor in which fluidized bed bodies having a mean diameter (number average) greater than the particle diameter of the dicarboxylic acid are used.

6. Process according to Claim 5, **characterized in that** the fluidized bed bodies have a solids density ρₛ of 0.5 g·cm⁻⁵ < ρₛ < 6 g·cm⁻³.

7. Process according to any of Claims 1 to 6, **characterized in that** the fluidized bed bodies are used as heat transfer media which are preheated outside the reaction zone and circulated through the reaction zone consist partly or entirely of a catalytically active material.

8. Process according to any of Claims 1 to 7, **characterized in that** the fluidized bed bodies consist partly or entirely of a catalytically active material, in particular copper or a copper salt, preferably CuCO₃.

9. Process according to any of Claims 1 to 8, **characterized in that** any solid carried out from the reaction zone by the gas stream is separated off from the product by means of a cyclone and/or filter.

10. Process according to any of Claims 1 to 9, **characterized in that** unreacted solid starting material separated off from the product gas steam is recirculated batchwise or continuously to the reaction zone.

## Revendications

1. Procédé de décarboxylation thermique d'acides dicarboxyliques dialkoxythiophéniques et alcylènedioxythiophéniques, en particulier de l'acide 3,4-éthylènedioxythiophène-2,5-dicarboxylique, en tant que produit de départ, **caractérisé en ce que** l'on utilise le produit de départ en tant que substance solide et/ou **en ce que** l'on effectue la réaction en présence d'une pluralité de corps de couches fluidisées, la réaction étant effectuée en l'absence de solvants et le produit de décarboxylation se formant lors de la réaction, en particulier le 3,4-éthylènedioxythiophène étant évacué sous forme gazeuse hors de la zone de réaction.

2. Procédé selon la revendication 1, **caractérisé en ce que** la décarboxylation est effectuée à une température de 100 à 600°C, de préférence, de 100 à 500°C, plus préférablement, de 150 à 400°C.

3. Procédé selon au moins l'une des revendications 1 à 2, **caractérisé en ce que** le procédé est effectué d'une manière continue dans une couche fluidisée formant des bulles ou turbulente ou traversée par des jets ou dans un lit fluidisé circulant de façon interne ou de façon externe.

4. Procédé selon au moins l'une des revendications 1 à 3, **caractérisé en ce que** la réaction est effectuée en présence d'un gaz auxiliaire inerte, en particulier sélectionné parmi la série gaz rares, azote, vapeur d'eau, monoxyde de carbone ou dioxyde de carbone ou d'un mélange de différents gaz auxiliaires inertes de ce genre.

5. Procédé selon l'une quelconque des revendications 1 à 4, **caractérisé en ce que** la réaction est effectuée dans un réacteur à couches fluidisées ou dans un réacteur à lit fluidisé, dans lequel on utilise des corps de couches fluidisées ayant un diamètre moyen (moyenne au nombre), qui est supérieur au diamètre des particules de l'acide dicarboxylique.

6. Procédé selon la revendication 5, **caractérisé en ce que** les corps de couches fluidisées présentent une densité de susbtances solides ρₛ telle que 0,5 g·cm⁻³ < ρₛ < 6 g·cm⁻³.

7. Procédé selon l'une quelconque des revendications 1 à 6, **caractérisé en ce que** l'on utilise, en tant qu'agents caloporteurs, des corps de couches fluidisées qui sont chauffés au préalable hors de la zone de réaction et qui sont circulés à travers la zone de réaction et qui se composent partiellement ou complètement d'un matériau actif du point de vue catalytique.

8. Procédé selon l'une quelconque des revendications 1 à 7, **caractérisé en ce que** les corps de couches fluidisées se composent partiellement ou complètement d'un matériau actif du point de vue catalytique, en particulier de cuivre ou d'un sel de cuivre, de préférence, de CuCO₃.

9. Procédé selon l'une quelconque des revendications 1 à 8, **caractérisé en ce que** l'on sépare la substance solide évacuée, le cas échéant, par le courant gazeux, hors de la zone de réaction à l'aide d'un cyclone et/ou d'un filtre.

10. Procédé selon l'une quelconque des revendications 1 à 9, **caractérisé en ce que** l'on reconduit à nouveau dans la zone de réaction, par charges ou en mode continu, la substance solide de départ n'ayant pas réagi, séparée du courant gazeux de produit.
